# FASCICULE DE BREVET EUROPEEN

(11) **EP 0 299 858 B1**
(45) Date de publication et mention de la délivrance du brevet: **06.10.1993**
(21) Numéro de dépôt: 88401785.6
(22) Date de dépôt: 08.07.1988
(51) Int. Cl.: A61F 2/08, A61L 27/00

(54) **Ligaments artificiels et articles permettant de les réaliser**
Bänder- und Sehnenprothese und Vorrichtung zur Herstellung derselben
Artificial ligament and apparatus for its manufacture

(30) Priorité: 09.07.1987 FR 8709776
(43) Date de publication de la demande: 18.01.1989
(73) Titulaire: CENDIS MEDICAL SARL, F-75014 Paris (FR)
(72) Inventeur: Lemaire, Marcel, F-60560 OrryLa Ville (FR)
(74) Mandataire: Hasenrader, Hubert

(56) Documents cités:
- EP-A- 0 051 954
- EP-A- 0 122 744
- WO-A-85/00511
- FR-A- 2 135 825
- FR-A- 2 278 313
- FR-A- 2 598 315

## Description

La présente invention concerne des ligaments artificiels et les articles permettant de réaliser lesdits ligaments.

On sait qu'un ligament est un ensemble de fibres conjonctives serrées et résistantes orientées reliant les os au niveau des articulations ou maintenant en place certains organes. Ces ligaments peuvent subir certaines lésions ou déchirures nécessitant soit leur renforcement, soit leur remplacement total ou partiel.

Pour effectuer ces diverses réparations, on a besoin de ligaments artificiels qui font l'objet de la présente invention.

Les ligaments artificiels selon la présente invention sont des objets de synthèse qui se différencient nettement des ligaments ou fragments ligamentaires que le chirurgien peut prélever sur le patient lui-même (auto-greffe) ou sur un patient compatible.

Il faut noter que le FR-A 2 135 825 décrit un produit destiné à la réalisation d'un ligament artificiel qui se présente sous la forme d'un voile obtenu par tricotage d'un fil en un matériau synthétique mécaniquement très résistant.

Il a été trouvé, et cela constitue l'une des idées centrales de la présente invention, qu'il était souhaitable de mettre à la disposition des chirurgiens confrontés avec des problèmes ligamentaires des articles qui, par manipulation simple, donnent naissance à un ligament ou à une portion de ligament.

La présente invention a pour but de répondre à ces besoins de manière satisfaisante.

Ce but est atteint au moyen d'un produit destiné à la réalisation d'un ligament artificiel qui se présente sous la forme d'un voile obtenu par tricotage d'un fil en un matériau synthétique mécaniquement très résistant caractérisé en ce que ledit produit est constitué uniquement d'un voile susceptible de donner naissance audit ligament par pliure, torsade ou roulage sur lui-même, ledit voile étant obtenu par tricotage de type RACHEL.

Bien entendu, ce morceau de voile devra posséder un certain nombre de propriétés dont certaines parviendront du matériau utilisé pour réaliser ce voile et dont d'autres proviendront du mode de fabrication du voile.

Le matériau utilisé pour réaliser le voile de l'invention peut-être n'importe quelle matière plastique que l'on peut mettre sous forme de fil, fibre ou filament, et qui présente alors une résistance mécanique élevée. Ainsi, les fibres utilisables sont celles qui présentent une ténacité, en grammes par denier, supérieure à environ 10. On citera notamment, dans cette famille, les fibres de polyamides aromatiques et les fibres en polyéthylène haute densité. Bien évidemment, ledit matériau devra être compatible avec les tissus vivants et il a été trouvé que le polyéthylène haute densité constituait de ce fait le matériau préféré pour la fabrication du voile. Il est possible également d'utiliser un matériau comportant d'une part des fibres de polyamides aromatiques et/ou de polyéthylène haute densité et, d'autre part, une portion plus faible (1 à 20% environ) de fibres de renforcement telles que des fibres métalliques, les fibres de bore ou les fibres de carbone par exemple.

Le mode de fabrication du voile doit être un mode de tricotage dit à "mailles jetées" tel qu'on peut l'obtenir par exemple sur les métiers de type Rachel. Un voile ainsi réalisé aura la propriété avantageuse de présenter une certaine élasticité dans un sens et une élasticité beaucoup plus faible dans le sens perpendiculaire. De plus ce mode de tricotage permet de réaliser des voiles présentant des ouvertures régulièrement réparties et visibles à l'oeil nu (c'est-à-dire dont les dimensions sont supérieures à environ 0,2mm et inférieures à 2mm) ce qui autorise une circulation de divers liquides à travers le ligament constitué.

Ainsi, selon l'invention, le chirurgien disposant du morceau de voile convenablement dimensionné obtenu par tricotage "Rachel" pourra, en repliant, torsadant ou enroulant ce morceau sur lui-même et en le fixant à l'état replié ou enroulé, obtenir le ligament ou la portion de ligament souhaitée. On conçoit d'ailleurs que, étant donné que le voile possède des propriétés différentes dans deux sens perpendiculaires (comme expliqué ci-dessus) le chirurgien en choisissant convenablement son mode de repliage, torsadage ou roulage pourra obtenir des ligaments présentant des propriétés différentes. On pourra encore modifier les propriétés du filament obtenu en pliant ou enroulant le voile autour d'une âme en un matériau fibreux très résistant, par exemple en fibres de carbone.

Mais il est également possible selon l'invention de réaliser ce pliage, cette torsade ou cet enroulement, sur lui-même, du morceau de voile dans une unité de production et de commercialiser ainsi des ligaments prêts à l'emploi.

L'exemple non limitatif ci-dessous.

On a réalisé par tricotage de type Rachel de fibres en polyéthylène haute densité (densité de la fibre 0,97, ténacité (g/denier) 30) un voile ayant une forme rectangulaire de 20 cm de long et de 10 cm de large. Ce voile a été enroulé sur lui-même pour former un cylindre de 20 cm de long et de 0,6 cm de diamètre. Ce cylindre est directement utilisable comme ligament artificiel pour le genou ; les deux extrémités dudit cylindre étant fixées par des moyens connus aux os convenables.

## Revendications

1. Produit destiné à la réalisation d'un ligament artificiel qui se présente sous la forme d'un voile obtenu par tricotage d'un fil en un matériau synthétique mécaniquement très résistant, caractérisé en ce que ledit produit est constitué uniquement d'un voile obtenu par tricotage de type RACHEL, et présentant une élasticité déterminée dans un sens et une élasticité beaucoup plus faible dans un sens perpendiculaire, ledit voile étant susceptible de donner naissance audit ligament par pliure, torsade ou roulage sur lui-même.

2. Produit selon la revendication 1, caractérisé en ce que ledit matériau synthétique contient du polyéthylène très haute densité.

3. Ligament, caractérisé en ce qu'il est obtenu par pliure ou roulage sur lui-même d'un produit selon la revendication 1.

## Claims

1. Product for producing an artificial ligament which is in the form of a web obtained by knitting a yarn in a mechanically very resistant synthetic material, characterized in that said product is solely constituted by a web obtained by the RACHEL-type knitting, and having a determined elasticity in one direction and an elasticity which is more reduced in a perpendicular direction, said web being capable of giving rise to said ligament by folding, twisting or rolling round itself.

2. Product according to claim 1, characterized in that said synthetic material contains polyethylene of very high density.

3. Ligament, characterized in that it is obtained by folding or rolling around itself a product according to claim 1.

## Patentansprüche

1. Produkt zur Herstellung eines künstlichen Bandes, das in der Form eines durch Stricken eines Garns aus mechanisch hochfestem synthetischem Werkstoff erhaltenen Vliesses ist, dadurch gekennzeichnet, dass besagtes Produkt ausschliesslich aus einem durch RACHEL-artiges Stricken erhaltenen Vliess besteht und in eine Richtung eine bestimmte Elastizität und in senkrechte Richtung dazu eine viel geringere Elastizität aufweist, wobei besagtes Vliess, dadurch dass es gefacht, gezwirnt oder auf sich gerollt wird, zum besagten Band führen kann.

2. Produkt nach Anspruch 1, dadurch gekennzeichnet, dass besagter synthetischer Werkstoff Polyethylen hoher Dichte enthält.

3. Band, dadurch gekennzeichnet, dass es durch Fachen oder Auf-Sich-Rollen eines Produktes nach Anspruch 1 erhalten wird.
